# EUROPÄISCHE PATENTANMELDUNG

(11) **EP 0 530 595 A1**
(43) Veröffentlichungstag der Anmeldung: **10.03.1993**
(21) Anmeldenummer: 92114174.3
(22) Anmeldetag: 20.08.1992
(51) Int. Cl.: A61B 17/34, A61M 25/06

(54) **Trokarhülse**

(30) Priorität: 03.09.1991 DE 4129237
(71) Anmelder: Richard Wolf GmbH, D-75438 Knittlingen (DE)
(72) Erfinder: Heckele, Helmut, W-7134 Knittlingen (DE); Heimberger, Rudolf, W-7519 Oberderdingen (DE)
(74) Vertreter: Wilcken, Thomas, Dipl.-Ing.

(57) **Zusammenfassung**

Die beschriebene Trokarhülse (1) ist in der Lage, starre gekrümmte Hilfsinstrumente (4) aufzunehmen und ist zumindest auf einem Teil ihrer Länge biegsam ausgebildet, so daß sich ihre Form durch elastisches Biegen, im übrigen aber knickfest den Krümmungen der Hilfsinstrumente (4) anzupassen vermag. Die Biegsamkeit und gleichzeitige Knickfestigkeit wird dabei durch Ausbildung der Trokarhülse (1) mit Vorsprüngen (12) an ihrem Außenumfang erzielt, die wendelförmig angelegt sein können und eine verbesserte Halterung der Tokarhülse (1) in der Punktionswunde gegen unbeabsichtigtes Herausgleiten bewirken.

## Beschreibung

Die Erfindung geht aus von einer Trokarhülse zum Durchführen von Hilfsinstrumenten.

Bei endoskopischen Eingriffen in Körperhöhlen, zu denen kein natürlicher Zugang besteht, z.B. zur Bauchhöhle, wird der Zugang durch einen Trokar mit einer Trokarhülse geschaffen. Hierzu wird ein scharfer Trokar zusammen mit der Trokarhülse, in welche dieser Trokar eingeführt ist, in den Körper des Patienten eingebracht. Der Torkar wird nach erfolgtem Einstich entfernt, während die Trokarhülse im Körper verbleibt und so einen Zugang zur Körperhöhle bildet. Durch die Trokarhülse kann dann das jeweils benötigte Instrument eingeführt und bei Bedarf gegen andere Instrumente ausgetauscht werden.

Dabei tritt häufig der Fall ein, daß starre, gebogene bzw. gekrümmte Hilfsinstrumente durch die Trokarhülse in die Körperhöhle eingeführt werden müssen, um die zu behandelnde Körperhöhlenstelle zu erreichen.

Um derartige Hilfsinstrumente durch die Trokarhülse hindurchschieben zu können, müßte diese einen Kanalquerschnitt aufweisen, der größer sein muß als zum Hindurchführen gerader Hilfsinstrumente erforderlich wäre. Eine solche Vergrößerung bringt jedoch eine erhöhte traumatische Wirkung mit sich, so daß das Streben dahingeht, den Querschnitt der Trokarhülse so klein wie möglich zu halten, um diesen nachteiligen Effekt zu minimieren.

Die Aufgabe der Erfindung besteht daher darin, eine Trokarhülse so auszubilden, daß durch ihren Kanal auch gekrümmte Hilfsinstrumente geführt werden können, ohne daß zu diesem Zweck der Durchmesser dieses Kanals größer ausgebildet werden muß als er üblicherweise zum Durchführen von geraden Hilfsinstrumenten erforderlich ist.

Diese Aufgabe wird erfindungsgemäß dadurch gelöst, das die Trokarhülse zumindest auf einem Teil ihrer Länge derart biegsam ausgebildet ist, daß sich ihre Form durch elastisches Biegen, im übrigen aber knickfest etwaigen Krümmungen von starren Hilfsinstrumenten anpaßt.

Durch eine derartige Ausbildung der Trokarhülse können gekrümmte, starre Hilfsinstrumente in die Trokarhülse eingeführt und durch sie hindurchgeführt werden, da sich die Torkarhülse ohne Vergrößerung des Innendurchmessers der Krümmung der starren Hilfsinstrumente anpaßt. Es ist daher lediglich zu beachten, daß etwaige starre Teile der Trokarhülse in ihrer Länge kurz genug gehalten werden, um ein Durchführen der gegrümmten Teile des Hilfsinstrumentes erreichen zu können.

Eine Ausführung der Trokarhülse mit ovalem Außenumfang hat dabei den Vorteil, daß eine flache Bauform erreicht wird und relativ enge Bereiche, beispielsweise zwischen benachbarten Rippen, problemlos durchstochen werden können.

Gemäß einer weiteren vorteilhaften Ausführung kann die Trokarhülse mehrere Längskanäle aufweisen, was ermöglicht, durch einen Kanal beispielsweise ein starres gekrümmtes Hilfsinstrument hindurchzuführen, während in dem anderen Kanal z.B. ein flexibles Hilfsinstrument eingeführt werden kann, dessen Verlauf dann zwangsläufig der Form bzw. dem Verlauf des starren gekrümmten Hilfsinstrumentes folgen wird. Nach einer anderen Konstellation kann durch einen mittleren Kanal der Trokar und nach dessen Entfernen ein gekrümmtes Hilfsinstrument eingeführt werden, während seitliche Kanäle z.B. als Meß- oder Spülkanäle genutzt werden können.

Nach einer weiteren Ausgestaltung der erfindungsgemäßen Trokarhülse kann deren Außenumfang mit Vorsprüngen zur Verbesserung der Formstabilität und zur Erhöhung der Reibung im Gewebedurchstich versehen sein. Hierdurch wird ein sicherer Halt der Trokarhülse im Körper des Patienten erreicht, da die axiale Verschiebbarkeit aufgrund der erhöhten Reibung gehemmt ist.

Dieser Effekt kann dadurch noch verbessert werden, daß die am Außenumfang der Trokarhülse befindlichen Vorsprünge durch eine wendelförmige Verdickung des Hülsenschaftes gebildet sind. Durch Anordnung einer Verstärkung in der Wandung des aus flexiblem Material, wie z.B. aus Silikonen, bestehenden Hülsenschaftes kann die Knickfestigkeit und Formstabilität so erhöht werden, daß enge Biegungen der Trokarhülse erreichbar sind, ohne daß die Gefahr einer Querschnittsverformung und/oder Knickung besteht. In diesem Sinne besonders wirksam ist eine Verstärkung aus in dem Schaftmaterial eingebetteten Ringen, die auch Bestandteil einer Wendel sein können.

Durch Verwendung von Material mit Formgedächniseigenschaften für den Aufbau der Trokarhülse lassen sich besondere Effekte erzielen. So kann die Ausbildung so vorgesehen werden, daß die Trokarhülse bei üblichen Umgebungstemperaturen, das heißt solchen Temperaturen, die deutlich unter der Körpertemperatur liegen, einen geraden Verlauf einnimmt bzw. nach Abkühlung auf Umgebungstemperatur aus einem gekrümmten Verlauf in den geraden zurückkehrt. Bei einer derartigen Ausführung wird die Trokarhülse einen durch ein starres gekrümmtes Hilfsinstrument aufgezwungenen gekrümmten Verlauf unter dem Einfluß der Körpertemperatur beibehalten, so daß nach Herausziehen eines solchen Hilfsinstrumentes auch flexible Hilfsinstrumente durch den bzw. einen der Kanäle eingeführt werden können, die dann dem gekrümmten Verlauf der Hülse folgen werden.

Die Verwendung von Polytetrafluorethylen zumindest für den biegsamen Teil der Trokarhülse ergibt gute Gleiteigenschaften an den inneren Wandungen der Kanäle und eine körperverträgliche Ausführung.

Die erfindungsgemäße Trokarhülse ist nachstehend anhand von in der Zeichnung dargestellten Ausführungsbeispielen näher erläutert. Es zeigen:

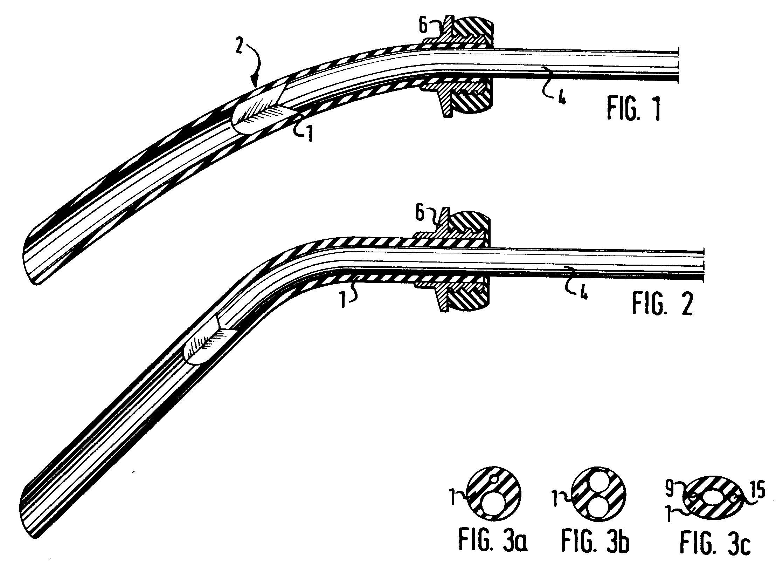

- Figur 1: einen Längsschnitt durch die Trokarhülse mit teilweise eingeführtem starren Hilfsinstrument,
- Figur 2: den gleichen Längsschnitt mit gegenüber Figur 1 weiter durch die Trokarhülse eingeführtem Hilfsinstrument,
- Figuren 3a bis 3c: drei Querschnitte durch unterschiedlich ausgebildete Trokarhülsen,
- Figur 4: einen Längsschnitt durch einen Teil der Trokarhülse mit einer Querschnittsverstärkung in Form von Ringen,
- Figur 5: einen Längsschnitt entsprechend Figur 4, eine andere Ausführung der Verstärkung zeigend,
- Figur 6: die Trokarhülse mit eingeführtem Trokar mit wendelförmig über die Schaftlänge verlaufenden Verstärkungen auf dem Umfang der Trokarhülse.

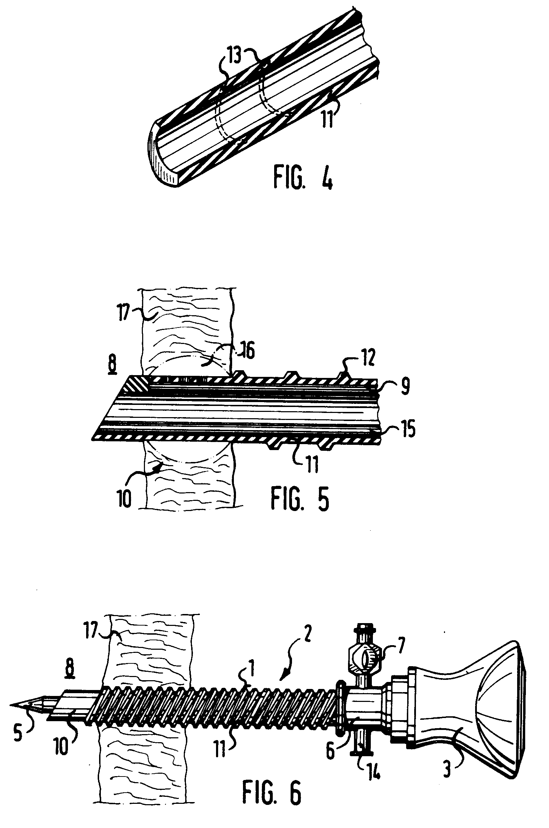

Gemäß Figur 6 besteht das erfindungsgemäße Trokarinstrument aus einer Trokarhülse 1 für die Führung eines Trokars, der im wesentlichen aus einer Handhabe 3 mit daran angebrachtem Dorn 4 besteht, der in durch die Trokarhülse 1 hindurchgeführter Position dargestellt ist und distalseitig in einer Spitze 5 endet.

Die Trokarhülse 1 weist ihrerseits proximalseitig ein Handhabenteil 6 auf, an welchem seitlich ein Hahn 7 angebracht ist, der beispielsweise über einen Schlauch mit einer Einrichtung zur Gasinsufflation oder mit einer Einrichtung zur Zufuhr von Flüssigkeit in eine Körperhöhle 8 versehen ist. Der Hahn 7 steht mit dem Hohlraum der Trokarhülse 1 oder mit einem in der Wandung derselben von der Handhabe 6 bis zum distalen Ende geführten Kanal 9 (Figur 5) in Verbindung, so daß über den Hahn 7 zugeführte Flüssigkeit oder Insufflationsgas in die Körperhöhle gelangen kann.

Das distale Endteil 10 ist biegesteif ausgeführt und mit dem Handhabenteil 6 über einen Hülsenteil 11 aus einem Werkstoff, z.B. einem Kunststoff, verbunden, der biegsam aber knickfest und querschnittssteif ausgebildet ist. Die Querschnittssteifigkeit wird dabei durch Vorsprünge oder Erhebungen 12 erreicht, die aus der Oberfläche des Hülsenteils 11 aufragen. Diese Erhebungen 12 können aus einzelnen, in die Wandung des Hülsenteils 11 eingebetteten Ringen 13 oder als Bestandteil einer Wendel ausgeführt sein und eine Art Außengewinde bilden, das sich dann über das Hülsenteil 11 erstreckt, wie das in Figur 5 angedeutet ist.

Wie weiter aus Figur 5 ersichtlich ist, kann der distale Endbereich der Trokarhülse 1 mit einem Ballonteil 16 ausgestattet sein, der über einen weiteren Kanal 15 mit einem weiteren Anschluß 14 an dem Handhabenteil 6 in Verbindung steht.

Das erfindungsgemäße Instrumentarium 2 wird wie folgt gehandhabt: Zunächst wird die Trokarhülse 1 zwecks Herstellung eines Punktionskanals mit einem Trokar bestückt und diese Einheit 2 sodann in das die zu behandelnde Körperhöhle 8 umgebende Gewebe 17, z.B. die Bauchdecke, eingestochen, bis das distale Trokarhülsenende genugend weit in die Körperhöhle 8 hineinragt.

Es kann auch so vorgegangen werden, daß zunächst die Einheit 2 so weit in das die zu behandelnde Körperhöhle 8 umgebende Gewebe 17 eingestochen wird, bis das gewindeartig ausgebildete Zwischenstück des Hülsenteils 11 an das zu durchdringende Gewebe 17 stößt. Dann wird die Trokarhülse 1 unter Drehbewegung gewissermaßen in das Gewebe eingeschraubt, bis das distale Trokarhülsenende genügend weit in die Körperhöhle 8 hineinragt.

Nach Entfernen des Trokars können durch die Trokarhülse 1 die jeweils benötigten Instrumente eingeführt werden, die bei der erfindungsgemäßen Ausführung auch starr gebogen sein können, da die Trokarhülse 1 sich dem Verlauf eines solchen Instrumentes anzupassen vermag. Nachdem dieses vollständig durch die Trokarhülse 1 hindurchgeführt ist, kann diese wieder ihren geraden Verlauf annehmen.

Aufgrund der Ausgestaltung der Oberfläche der Trokarhülse 1 wird ein unbeabsichtigtes Herausgleiten, z.B. beim Instrumentenwechsel, weitgehend vermieden. Eine zusätzliche Sicherung läßt sich mit der Ausbildung nach Figur 5 erreichen, indem der Ballonteil 16 durch Zufuhr von Flüssigkeit oder Gas durch den Kanal 15 hindurch aufgebläht wird, nachdem die Trokarhülse 1 genügend weit durch das Gewebe 17 in die Körperhöhle eingeführt wurde. Der Ballonteil 16 wird dadurch gegen das umgebende Gewebe 17 gepreßt, so daß ein versehentliches Herausgleiten der Trokarhülse 1 aus der Punktionswunde ausgeschlossen ist.

## Patentansprüche

1. Trokarhülse (1) zum Durchführen von Hilfsinstrumenten, dadurch gekennzeichnet, daß sie zumindest auf einem Teil ihrer Länge derart biegsam ausgebildet ist, daß sich ihre Form durch elastisches Biegen, im übrigen aber knickfest etwaigen Krümmungen von starren Hilfsinstrumenten (4) anpaßt.

2. Trokarhülse nach Anspruch 1, dadurch gekennzeichnet, daß sie einen ovalen Außenumfang aufweist.

3. Trokarhülse nach einem der Ansprüche 1 und 2, dadurch gekennzeichnet, daß sie mehrere Längskanäle (9,15) aufweist.

4. Trokarhülse nach einem der Ansprüche 1 bis 3, dadurch gekennzeichnet, daß ihr Außenumfang mit Vorsprüngen (12) zur Verbesserung der Formstabilität und zur Erhöhung der Reibung im Gewebedurchstich versehen ist.

5. Trokarhülse nach Anspruch 4, dadurch gekennzeichnet, daß die Vorsprünge (12) durch eine wendelförmige Verdickung des Hülsenschaftes (11) gebildet sind.

6. Trokarhülse nach einem der Ansprüche 1 bis 5, dadurch gekennzeichnet, daß in der Wandung des aus flexiblem Material bestehenden Hülsenschaftes (11) eine dessen Knickfestigkeit und Formstabilität erhöhende Verstärkung vorgesehen ist.

7. Trokarhülse nach Anspruch 6, dadurch gekennzeichnet, daß die Verstärkung aus im Schaftmaterial eingebetteten Ringen (13) besteht.

8. Trokarhülse nach Anspruch 6, dadurch gekennzeichnet, daß die Verstärkung aus einer im Schaftmaterial eingebetteten Wendel besteht.

9. Trokarhülse nach einem der Ansprüche 1 bis 7, dadurch gekennzeichnet, daß sie aus Material mit Formgedächtniseigenschaften besteht.

10. Trokarhülse nach einem der Ansprüche 1 bis 9, dadurch gekennzeichnet, daß der Hülsenschaft aus Polytetrafluorethylen besteht.

11. Trokarhülse nach einem der Ansprüche 1 bis 9, dadurch gekennzeichnet, daß der Hülsenschaft aus Silikonen besteht.
